# EUROPEAN PATENT APPLICATION

(11) **EP 4 414 360 A1**
(43) Date of publication of application: **14.08.2024**
(21) Application number: 23156097.0
(22) Date of filing: 10.02.2023
(51) Int. Cl.: C07C 231/24, C07C 231/02, C07C 233/47

(54) **METHOD FOR PURIFICATION OF AMINO ACID SURFACTANTS**

(71) Applicant: BASF SE, 67056 Ludwigshafen am Rhein (DE)
(72) Inventor: THERRE, Jörg, 67056 Ludwigshafen am Rhein (DE); GODIN, Alexandra, 40589 Düsseldorf (DE); MAHNKE, Eike Ulf, 40589 Düsseldorf-Holthausen (DE); ALTUNOK, Mehmet Yuecel, 40589 Düsseldorf-Holthausen (DE); RATHS, Hans-Christian, 33824 Werther (DE)
(74) Representative: BASF IP Association

(57) **Abstract**

The present invention relates to a method for producing a purified amino acid surfactant from a raw amino acid surfactant containing impurities, comprising the steps of
(i) providing the raw amino acid surfactant; and
(ii) subjecting the raw amino acid surfactant to an ultrafiltration stage; and
(iii) collecting the so produced purified amino acid surfactant,
wherein the ultrafiltration stage comprises a filter membrane having a separation limit molecular cut-off from 1000 g/mol to 100,000 g/mol, preferably from 4000 g/mol to 50,000 g/mol, more preferably from 5000 g/mol to 30,000 g/mol.

The method can produce purified amino acid surfactants with very low levels of impurities, notably inorganic salts, residual amino acid and polar organic solvents. Furthermore, the method provides high filtration rates with a permeate relatively low in polar organic solvent content.

## Description

### Field of the Invention

The present invention concerns a method for providing a purified amino acid surfactant from a raw amino acid surfactant that contains impurities. Such impurities arise out of the manufacturing process of amino acid surfactants and tend to include residual amino acid, side products, such as inorganic salts and polar organic solvents. The method developed by the inventors involves ultrafiltration of the raw amino acid surfactant using filter membranes having a specific range of separation limit molecular weight cut-off.

### Background of the Invention

Amino acid surfactants are typically long chain acyl N-substituted amino acids. They are regarded as biocompatible and biodegradable surfactants and found to be useful in a variety of applications such as personal care applications and biomedical applications. Amino acid surfactants have been used as preservatives in for instance drugs and cosmetics, having antibacterial, antiviral and anti-yeast properties. Common applications for amino acid surfactants include skin care, hair cleaning, dental products including shampoos, body washes, shower gels, cosmetics, toothpaste and toiletries. Other applications can include products in the fields of biology and medicine; emulsifiers, defoamers and cleaning agents in food applications; products in the agricultural field; products for mineral flotation and petroleum fields; bactericides; dispersants; and detergents.

Conventionally amino acid surfactants are usually produced by the reaction of an amino acid, typically as an alkali metal salt, such as the sodium salt, and a long chain acyl halide, suitably a long chain acyl chloride in the presence of water and a polar organic solvent. Usually, a molar excess of amino acid would be used in the reaction to ensure complete consumption of the acyl halide. The reaction product, which may be regarded as a raw amino acid surfactant, contains impurities resulting from the reaction. This will include inorganic salt, such as NaCl, produced as a by-product; residual amino acid; and polar solvent, such as isopropanol or 1 ,2-propanediol. These impurities need to be removed or at least reduced to acceptable levels in order for the amino acid surfactant to meet the standards for application.

One conventional method for separating the impurities from the amino acid surfactant involves combining concentrated hydrochloric acid with the raw amino acid surfactant to separate out the soluble impurities, notably NaCl, polar solvent and amino acid and some of the water and forming amino acid surfactant in insoluble acid form as precipitated solids. The solids need to be washed extensively with water and then filtered by an appropriate device. This is followed by treatment with sodium hydroxide to yield the purified amino acid surfactant as the sodium salt. This process has the disadvantage that concentrated mineral acids such as concentrated hydrochloric acid are highly corrosive. Thus, additional safety measures for the safe handling of the concentrated acid and the use of expensive hydrochloric acid resistant reactors are a prerequisite for this procedure.

An alternative process of purifying the raw amino acid surfactant requires first acidifying the reaction mixture, typically with concentrated hydrochloric acid, to form two phases with one phase containing the inorganic salt, i.e. NaCl, a small amount of the polar solvent, for instance isopropanol or 1,2-propanediol, and some of the water and the other phase containing the amino acid surfactant. This is subjected to phase separation of the product phase and the phase containing the water-soluble byproducts. The product phase containing the amino acid surfactant is further treated with water before subjecting the mixture to distillation at high temperature to remove the polar solvent and leaving the purified amino acid surfactant. This method also has the aforesaid disadvantages of using concentrated hydrochloric acid. In addition, the phase separation method requires high amounts of polar solvent to be used during the reaction. This polar solvent partially remains in the amino acid surfactant product and hence must be removed by distillation. This additional thermal stress during the distillation stage can result in partial degradation of the product and the formation of soap, amino acid salt, pyroglutamates and/or solvent esters. A further disadvantage is that during the washing procedure some amino acid surfactant product is lost during the removal of the water.

Japanese patent application JP07002747 A (06 June 1995) describes a method for purifying an N-long chain acyl acidic amino acid salt. The method involves condensing and acidic amino acid and long chain fatty acid halide having a saturated or unsaturated acyl group having 8 to 22 carbon atoms in the presence of an alkali at a pH of 8 to 13.5 in a solution containing a mixture of water and hydrophilic organic solvent. A membrane separation process is said to be used to obtain a high purity N-long chain acyl acidic amino acid salt. The Examples describe neutralising the mixture with hydrochloric acid and adjusting the pH to between 7.0 and 8.0, adding water and then removing impurities using a polyamide-based ultrafiltration membrane having a molecular weight cut-off of 200 to 500 at an operation pressure of 10 to 20 kg/cm², at a temperature of 20 to 30°C and a feed water flow of 13 to 15 L/m² · hour for 2 hours. The amount of inorganic salt contained in the product was 0.1% or less.

A review by Jing Guo et al., "Review: Progress in synthesis, properties and application of amino acid surfactants", Chemical Physics Letters 794 (2022) 139499 provides an overview of amino acid surfactants categories, synthesis and applications in various sectors. Amino acid surfactants are said to have physio chemical properties that include surface activity, aggregate, gel, biodegradation and having applications in daily chemical, biological medicine, food, agriculture and industry.

It is an objective of the present invention to provide a more efficient method of purifying amino acid surfactants. A further objective is in the development of such a purification method that avoids the use of strong mineral acids such as hydrochloric acid. The inventors also sought to avoid a filtration process requiring high pressure and low flow rates which on an industrial scale would tend to require a large and expensive filtration systems with a large membrane area. In addition, the invention addresses the need to avoid a method that risks producing high levels of hazardous wastewater. The invention also addresses the need for a purification method which can produce highly pure amino acid surfactants, particularly with very low levels of impurities such as inorganic salts and/or residual amino acid and/or organic solvents.

### Summary of the Invention

The present invention defines a method for producing a purified amino acid surfactant from a raw amino acid surfactant containing impurities, comprising the steps of
(i) providing the raw amino acid surfactant; and
(ii) subjecting the raw amino acid surfactant to an ultrafiltration stage; and
(iii) collecting the so produced purified amino acid surfactant,
wherein the ultrafiltration stage comprises a filter membrane having a separation limit molecular cut-off from 1000 g/mol to 100,000 g/mol, preferably from 4000 g/mol to 50,000 g/mol, more preferably from 5000 g/mol to 30,000 g/mol.

### Description of Drawings

Figure 1 is a diagram of a high-pressure stirred cell used for the amino acid surfactant Raw SG1 purification experiments of Examples 1-11.
Figure 2 is a diagram of the pilot plant unit used for the amino acid surfactant Raw SG2 purification experiments of Examples 12-16.

### Detailed Description of the Invention

The inventive method enables highly pure amino acid surfactants to be conveniently produced addressing the aforementioned objectives. Specifically, this method avoids the necessity to handle strong mineral acids, provides a process which can be scaled up to an industrial level producing commercial quantities of product without the need for large expensive filtration systems occupying a large footprint. In particular, the method enables purified amino acid surfactants to be produced with extremely low levels of inorganic salts, residual amino acids and organic solvents without producing high levels of hazardous wastewater.

The raw amino acid surfactant would generally be produced by the reaction of an amino acid (including alkali metal salts thereof) with an acyl halide in an alkaline medium containing a polar organic solvent. The alkaline medium would normally fully neutralise the amino acid to produce a fully neutralised alkali metal salt of the amino acid.

The term raw amino acid surfactant means the unpurified amino acid surfactant resulting from the manufacturing process containing the impurities. Typically, the impurities contained in the raw amino acid surfactant would normally comprise an inorganic salt, a polar organic solvent and optionally an amino acid. The inorganic salt would normally be the salt of an alkali metal and halide, for instance sodium chloride. The inorganic salt therefore results as a by-product from the condensation reaction of the acyl halide and the alkali salt of the amino acid. The inorganic salt resulting depends on the alkali employed and the halide of the acyl halide. Desirably the alkali may be sodium hydroxide and the acyl halide may be an acyl chloride thus giving rise to sodium chloride as the inorganic salt. The polar organic solvent may be any of suitable polar organic solvents in which the amino acid and amino acid surfactant would be soluble or at least miscible. Desirably, the polar solvent may be any hydrophilic solvent which is miscible with water. A suitable polar solvent should also be essentially inert to the acyl halide, typically acyl chloride, used in the reaction to produce the amino acid surfactant. Indicative examples of suitable polar organic solvents include methanol, ethanol, isopropanol, 1-propanol, tert-butanol, sec-butanol, iso-butanol, n-butanol, ethylene glycol, 1,2-propanediol, 1,3-propanediol, glycerol, acetone, methyl ethyl ketone, tetrahydrofuran, cyclohexanone, 1,4-dioxane, etc. but preferably is isopropanol or 1,2-propanediol. Normally the amino acid should be used in a molar excess of the acyl halide in order to ensure that all of the acyl halide is consumed. As a result of this the raw amino acid surfactant is likely to contain an excess of the unreacted amino acid.

Thus, the raw amino acid surfactant would normally result from a process that precedes the inventive method for producing the purified amino acid surfactant.

Such a process may be generally summarised as a process for preparing a raw amino acid surfactant by reacting an amino acid, (including an alkali metal salt thereof) with an acyl halide in an aqueous alkaline medium containing a polar organic solvent. Preferably the amino acid would be used in a molar excess of the acyl halide.

In a desirable embodiment of the invention the raw amino acid surfactant is comprised in an aqueous medium. Preferably the aqueous medium should have a pH in a range which avoids precipitation.

Desirably the raw material amino acid surfactant is comprised in an aqueous medium, preferably which aqueous medium has a pH sufficient for at least one of the acid groups of the amino acid surfactant to be in the salt form. In the case of polyprotic amino acid surfactants, for instance diprotic amino acid surfactants, it should be sufficient for the aqueous medium to be at a pH at which only one of the acid groups is in the salt form.

Suitably the pH of the aqueous medium may lie in the range from greater than 4.0 to 14.0, from 5.0 to 14.0, for instance from 6.0 to 13.5, from 7.0 to 13.0, from 8.0 to 12.5, from 8.5 to 12.0, from 8.5 to 11.5. The exact desired pH will depend on the particular amino acid surfactant in the aqueous medium.

For stearoyl glutamate it is preferred that the aqueous medium has a pH of greater than 8.0, for instance greater than 8.0 to 13.5, for instance from 8.1 to 13.0, desirably from 8.2 to 12.5, more desirably from 8.3 to 12.0, suitably from 8.3 to 11.5, from 8.3 to 11.0, from 8.5 to 11.0, from 9.0 to 11.0.

In this preferred embodiment the method avoids the risk of forming globules or agglomerates. This can be a particular problem at lower pHs. In the case of stearoyl glutamate a pH below 8.0 has been found to be problematic in regard to the formation of globules or agglomerates.

The avoidance of such globules or agglomerates should reduce the risk of blinding filter membranes.

Desirably the raw amino acid surfactant should be diluted with an aqueous dilution medium. The aqueous dilution medium may be any suitable aqueous medium effective for providing sufficient dilution to the raw amino acid surfactant. Preferably the aqueous dilution medium is water.

Preferably the raw amino acid surfactant should be diluted with an aqueous dilution medium by the amount of from 1:10 to 3:1, based on the weight of the raw amino acid surfactant to the weight of the aqueous dilution medium. More preferably the dilution can be from 1:10 to 1:1, more preferably from 1:6 to 1:1. The amount the raw amino acid surfactant is diluted may depend on the initial concentration or solids content of the raw amino acid surfactant. The initial solids content of the raw amino acid surfactant before diluting may for instance be as high as 50% w/w, typically, from 15% to 50%, from 20% to 40%.

The ultrafiltration stage may employ any of a variety of membranes for the method. Suitable filter membranes may be constructed from a variety of materials. One category of filter membranes includes polymeric membranes. Examples of polymeric membranes include symmetric (polymeric) membranes which possess a uniform (pore) structure over the thickness of the membrane. Alternatively, asymmetrical (polymeric) membranes may be used. Typically, filtration membranes may be formed from polyethylene, polyvinylidene fluoride (PVDF), polytetrafluoroethylene (PTFE), polyacrylonitrile, polyethersulfone, hydrophilized polyethersulfone or any combinations, for instance as composites. Other polymeric filter membranes include polyester and polycarbonate membranes which can be made using irradiation and etching processes from polymers such as polypropylene, polyamides, cellulose acetate, polyethersulfone and polysulfone. The polymeric membranes can be used as tubular membranes or flat sheet membranes. Preferred the polymeric membranes are used as flat sheet spiral wound membrane modules.

Other suitable filtration membranes may be constructed from inorganic materials, such as ceramics and metals. Suitable commercial ceramic membranes may be made by slip casting processes. Typically, this consists of 2 steps and begins with preparation of a dispersion of fine particles (referred to as slip) followed by deposition of the particles on a porous support. Inorganic membranes that are commonly available and suitable for the present invention include composites containing a thin separation barrier on a support (e.g. ceramic materials, such as titania, zirconia or alumina or combinations thereof). The ceramic and metals membranes can be used as tubular membranes or flat sheet membranes. Preferred the ceramic membranes are used as tubular multichannel ceramic membrane elements.

Preferred filter membranes are constructed from any of the materials selected from the group consisting of polyacrylonitrile, polyethersulfone, hydrophilized polyethersulfone and any combinations of these materials.

The ultrafiltration stage may be applied in a cross flow or dead-end mode. In a preferred embodiment the ultrafiltration stage comprises crossflow filtration.

Preferably the raw amino acid surfactant is in an aqueous medium, preferably at a pH greater than 4.0. As previously stated, the precise pH required will depend upon the particular amino acid surfactant. Suitably the pH should be sufficient for at least one of the acid groups of the amino acid surfactant to be in the salt form. The ultrafiltration preferably employs crossflow filtration. Typically, the aqueous medium containing the raw amino acid surfactant flows tangentially across the surface of the filter membrane. Desirably, when using spiral wound modules, the surface velocity of the aqueous medium is in the range from 0.1 to 1.0 m/s, preferably from 0.2 to 0.7 m/s and more preferably from 0.3 to 0.5 m/s. When using tubular modules, the surface velocity of the aqueous medium is in the range from 0.1 to 10.0 m/s, preferably from 0.5 to 7 m/s and more preferably from 1 to 5 m/s. During the process a proportion of the aqueous medium passes through the filter membrane as a permeate or filtrate and the remainder of the aqueous medium which does not pass through the filter membrane is termed a retentate. The permeate should contain a higher proportion of the soluble impurities such as the inorganic salt, polar organic solvent and optionally the residual amino acid. The retentate should contain a high proportion of the amino acid surfactant. The retentate can be recirculated in a recirculation loop and combined with further in coming aqueous medium containing the raw amino acid surfactant and then be passed through the crossflow filtration stage again.

In a preferred embodiment the raw amino acid surfactant is comprised in an aqueous medium which is flowed through the ultrafiltration stage forming a permeate and a retentate, in which impurities are removed through the permeate and amino acid surfactant is preferentially retained in the retentate, which retentate is recirculated in a recirculation loop.

Suitable average total flux across the membrane filter may be from 5 to 90 kg/m²/h or higher, for instance from 10 to 80 kg/m²/h or from 10 to 70 kg/m²/h. The desired average total flux across the membrane filter selected can depend upon many factors affecting the particular process plant employed and its operation.

The ultrafiltration stage suitably requires pressure applied to the aqueous medium comprising the raw amino acid surfactant to facilitate flow across the filter membrane. Normally the average trans-membrane pressure (TMP) would be at least 1.0 bar, typically at least 5.0 bar, The average trans-membrane pressure (TMP) may be as high as 90 bar, but generally would be no more than 50 bar, for instance up to 30 bar. Examples of suitable average trans-membrane pressure (TMP) ranges include from 1.0 to 30.0 bar, from 5.0 to 90.0 bar,

The aqueous medium in the recirculation loop and ultrafiltration stage would normally be at a temperature higher than ambient to retain sufficient fluidity of the aqueous medium and its components. Suitably the aqueous medium would be at a temperature from 10°C to 95°C, from 25°C to 90°C, from 30°C to 70°Cand preferably from 35°C to 60°C.

In one embodiment,the raw amino acid surfactant comprised in the aqueous medium is subjected to at least one diafiltration step. Typically, in the diafiltration step a diafiltration medium, which can be and usually is the same as the above-mentioned aqueous dilution medium, is fed into the recirculation loop at substantially the same rate as permeate flows from the ultrafiltration stage. Thus, in diafiltration mode the diafiltration medium (or aqueous dilution medium) entering the recirculation loop and permeate leaving the ultrafiltration stage at substantially the same rate means that the volume and concentration of the amino acid surfactant would remain substantially the same. Diafiltration may thus be regarded as a type of washing phase for the raw amino acid surfactant.

In an alternative embodiment, the at least one diafiltration step may be avoided by employing a high initial dilution of the raw amino acid surfactant. This would need to be followed by at least one concentration step to reduce the solids content to a desired level. Thus, in this alternative embodiment an analogous washing phase for the raw amino acid surfactant should be achieved.

The diafiltration factor (DF) is defined as the mass of the diafiltration medium used divided by the mass of the retentate. Preferably the method employs at least one diafiltration step and has an overall diafiltration factor (DF) from 1 to 10, preferably from 2 to 6.

In another more preferred embodiment, the raw amino acid surfactant in the aqueous medium is subjected to at least one concentration step. The concentration step normally involves no or substantially no dilution medium being fed into the recirculation loop while permeate is removed at the ultrafiltration stage or aqueous dilution medium being fed into the recirculation loop at a lower rate than the rate of permeate that flows from the ultrafiltration stage.

The number of times more concentrated the aqueous medium comprising the raw amino acid surfactant becomes during the concentration step is termed the concentration factor (CF). The concentration factor (CF) is defined as the mass of the retentate at the start of the concentration step divided by the mass of the retentate at the end of the concentration step. A concentration factor (CF) of exactly 1 means that the aqueous medium has not been concentrated. Thus, a concentration factor (CF) which is any number greater than 1 means that the aqueous medium has become more concentrated by that CF value.

More preferably, the raw amino acid surfactant in the aqueous medium is subjected to at least one of a diafiltration step and/or at least one of a concentration step. Particularly preferably, the aqueous medium would be subjected to a combination of diafiltration and concentration, carried out in sequence.

More preferably still, the aqueous medium comprising the raw amino acid surfactant is subjected to one or more steps of diafiltration and/or concentration which comprises the steps
a) optionally initial diafiltration (D1);
b) optional initial concentration (C1);
c) optional diafiltration (D2); and
d) optional final concentration (C2),
provided that at least one of steps a or c and/or at least one of steps b or d are included.

Especially preferably, the aqueous medium comprising the raw amino acid surfactant is subjected to a series of diafiltration and/or concentration which comprises in sequence
a) optionally initial diafiltration (D1);
b) optional initial concentration (C1);
c) optional diafiltration (D2); and
d) optional final concentration (C2),
provided that at least one of steps a or c and at least one of steps b or d are included.

More preferably still, the combination of diafiltration and concentration steps includes in sequence at least steps b), c) and d). In another preferred aspect, the combination of diafiltration and concentration steps includes in sequence at least steps a), b), c) and d).

Where the initial diafiltration (D1) step is employed, it is desirable that this is carried out with a diafiltration factor (DF1) from 0.01 to 3.0. Preferably the initial diafiltration (D1) is carried out with a diafiltration factor (DF1) from 0.3 to 1.0.

In a preferred form of the invention where the initial concentration (C1) step is carried out it is preferred that this would have a concentration factor (CF1) from 1.01 to 10.0 and preferably from 1.3 to 2.0.

Preferably the diafiltration (D2) step is employed and this would be subsequent to the initial concentration (C1) step. Desirably diafiltration (D2) step is carried out with a diafiltration factor (DF2) from 0.01 to 10.0, preferably from 1.5 to 5.0.

Preferably the method employs a final concentration (C2) step which is normally carried out after the diafiltration (D2) step. The final concentration (C2) step would normally be carried out with a concentration factor (CF2) from 1.1 to 4.0, preferably from 1.2 to 3.0.

The method for producing the purified amino acid surfactant may be continuous, batch, semi-batch or fed batch. Preferably the method is carried out in batch or fed batch operation. Desirably the fed batch process would involve the raw amino acid surfactant in an aqueous medium being placed in the recirculation loop, for instance a dilution vessel, and aqueous dilution medium, aqueous diafiltration medium and/or retentate being fed into the recirculation loop, for instance the dilution vessel, at a defined rate.

It is normally preferred that the amino acid surfactant is in the form of an alkali metal salt or an ammonium salt. The alkali metal salt may for instance be the sodium salt or the potassium salt, but preferably the amino acid surfactant is in the form of the sodium salt.

Preferably the amino acid surfactant is an N-substituted C₈-C₃₀-acyl amino acid, preferably N-substituted C₁₂-C₂₀-acyl amino acid, including the alkali metal salts thereof, for instance the sodium salt. The C₈-C₃₀-acyl moiety may be saturated or unsaturated.

Examples of suitable N-substituted C₈-C₂₀-acyl amino acids include the following compounds: N-capryloyl glycine, N-pelargonoyl glycine, N-capric glycine, N-n-undecanoyl glycine, N-lauroyl glycine, N-n-tridecanoyl glycine, N-myristoyl glycine, N-n-pentadecanoyl glycine, N-palmitoyl glycine, N-margaroyl glycine, N-stearoyl glycine, N-n-nonadecanoyl glycine, N-arachidoyl glycine, N-α-linolenoyl glycine, N-stearidonoyl glycine, N-eicosapentaenoyl glycine, N-linoleoyl glycine, N-γ-linolenoyl glycine, N-dihomo-γ-linolenoyl glycine, N-arachidonoyl glycine, N-behenoyl glycine, N-erucoyl glycine, N-capryloyl sarcosine, N-pelargonoyl sarcosine, N-capric sarcosine, N-n-undecanoyl sarcosine, N-lauroyl sarcosine, N-n-tridecanoyl sarcosine, N-myristoyl sarcosine, N-n-pentadecanoyl sarcosine, N-palmitoyl sarcosine, N-margaroyl sarcosine, N-stearoyl sarcosine, N-n-nonadecanoyl sarcosine, N-arachidoyl sarcosine, N-α-linolenoyl sarcosine, N-stearidonoyl sarcosine, N-eicosapentaenoyl sarcosine, N-linoleoyl sarcosine, N-γ-linolenoyl sarcosine, N-dihomo-γ-linolenoyl sarcosine, N-arachidonoyl sarcosine, N-behenoyl sarcosine, N-erucoyl sarcosine, N-capryloyl glutamic acid, N-pelargonoyl glutamic acid, N-capric glutamic acid, N-n-undecanoyl glutamic acid, N-lauroyl glutamic acid, N-n-tridecanoyl glutamic acid, N-myristoyl glutamic acid, N-n-pentadecanoyl glutamic acid, N-palmitoyl glutamic acid, N-margaroyl glutamic acid, N-stearoyl glutamic acid, N-n-nonadecanoyl glutamic acid, N-arachidoyl glutamic acid, N-α-linolenoyl glutamic acid, N-stearidonoyl glutamic acid, N-eicosapentaenoyl glutamic acid, N-linoleoyl glutamic acid, N-γ-linolenoyl glutamic acid, N-dihomo-γ-linolenoyl glutamic acid, N-arachidonoyl glutamic acid, N-behenoyl glutamic acid, N-erucoyl glutamic acid, N-capryloyl alanine, N-pelargonoyl alanine, N-capric alanine, N-n-undecanoyl alanine, N-lauroyl alanine, N-n-tridecanoyl alanine, N-myristoyl alanine, N-n-pentadecanoyl alanine, N-palmitoyl alanine, N-margaroyl alanine, N-stearoyl alanine, N-n-nonadecanoyl alanine, N-arachidoyl alanine, N-α-linolenoyl alanine, N-stearidonoyl alanine, N-eicosapentaenoyl alanine, N-linoleoyl alanine, N-γ-linolenoyl alanine, N-dihomo-γ-linolenoyl alanine, N-arachidonoyl alanine, N-behenoyl alanine, N-erucoyl alanine, N-capryloyl arginine, N-pelargonoyl arginine, N-capric arginine, N-n-undecanoyl arginine, N-lauroyl arginine, N-n-tridecanoyl arginine, N-myristoyl arginine, N-n-pentadecanoyl arginine, N-palmitoyl arginine, N-margaroyl arginine, N-stearoyl arginine, N-n-nonadecanoyl arginine, N-arachidoyl arginine, N-α-linolenoyl arginine, N-stearidonoyl arginine, N-eicosapentaenoyl arginine, N-linoleoyl arginine, N-γ-linolenoyl arginine, N-dihomo-γ-linolenoyl arginine, N-arachidonoyl arginine, N-behenoyl arginine, N-erucoyl arginine, N-capryloyl aspartic acid, N-pelargonoyl aspartic acid, N-capric aspartic acid, N-n-undecanoyl aspartic acid, N-lauroyl aspartic acid, N-n-tridecanoyl aspartic acid, N-myristoyl aspartic acid, N-n-pentadecanoyl aspartic acid, N-palmitoyl aspartic acid, N-margaroyl aspartic acid, N-stearoyl aspartic acid, N-n-nonadecanoyl aspartic acid, N-arachidoyl aspartic acid, N-α-linolenoyl aspartic acid, N-stearidonoyl aspartic acid, N-eicosapentaenoyl aspartic acid, N-linoleoyl aspartic acid, N-γ-linolenoyl aspartic acid, N-dihomo-γ-linolenoyl aspartic acid, N-arachidonoyl aspartic acid, N-behenoyl aspartic acid, N-erucoyl aspartic acid, N-capryloyl glutamine, N-pelargonoyl glutamine, N-capric glutamine, N-n-undecanoyl glutamine, N-lauroyl glutamine, N-n-tridecanoyl glutamine, N-myristoyl glutamine, N-n-pentadecanoyl glutamine, N-palmitoyl glutamine, N-margaroyl glutamine, N-stearoyl glutamine, N-n-nonadecanoyl glutamine, N-arachidoyl glutamine, N-α-linolenoyl glutamine, N-stearidonoyl glutamine, N-eicosapentaenoyl glutamine, N-linoleoyl glutamine, N-γ-linolenoyl glutamine, N-dihomo-γ-linolenoyl glutamine, N-arachidonoyl glutamine, N-behenoyl glutamine, N-erucoyl glutamine, N-capryloyl histidine, N-pelargonoyl histidine, N-capric histidine, N-n-undecanoyl histidine, N-lauroyl histidine, N-n-tridecanoyl histidine, N-myristoyl histidine, N-n-pentadecanoyl histidine, N-palmitoyl histidine, N-margaroyl histidine, N-stearoyl histidine, N-n-nonadecanoyl histidine, N-arachidoyl histidine, N-α-linolenoyl histidine, N-stearidonoyl histidine, N-eicosapentaenoyl histidine, N-linoleoyl histidine, N-γ-linolenoyl histidine, N-dihomo-γ-linolenoyl histidine, N-arachidonoyl histidine, N-behenoyl histidine, N-erucoyl histidine, N-capryloyl isoleucine, N-pelargonoyl isoleucine, N-capric isoleucine, N-n-undecanoyl isoleucine, N-lauroyl isoleucine, N-n-tridecanoyl isoleucine, N-myristoyl isoleucine, N-n-pentadecanoyl isoleucine, N-palmitoyl isoleucine, N-margaroyl isoleucine, N-stearoyl isoleucine, N-n-nonadecanoyl isoleucine, N-arachidoyl isoleucine, N-α-linolenoyl isoleucine, N-stearidonoyl isoleucine, N-eicosapentaenoyl isoleucine, N-linoleoyl isoleucine, N-γ-linolenoyl isoleucine, N-dihomo-γ-linolenoyl isoleucine, N-arachidonoyl isoleucine, N-behenoyl isoleucine, N-erucoyl isoleucine, N-capryloyl leucine, N-pelargonoyl leucine, N-capric leucine, N-n-undecanoyl leucine, N-lauroyl leucine, N-n-tridecanoyl leucine, N-myristoyl leucine, N-n-pentadecanoyl leucine, N-palmitoyl leucine, N-margaroyl leucine, N-stearoyl leucine, N-n-nonadecanoyl leucine, N-arachidoyl leucine, N-α-linolenoyl leucine, N-stearidonoyl leucine, N-eicosapentaenoyl leucine, N-linoleoyl leucine, N-γ-linolenoyl leucine, N-dihomo-γ-linolenoyl leucine, N-arachidonoyl leucine, N-behenoyl leucine, N-erucoyl leucine, N-capryloyl lysine, N-pelargonoyl lysine, N-capric lysine, N-n-undecanoyl lysine, N-lauroyl lysine, N-n-tridecanoyl lysine, N-myristoyl lysine, N-n-pentadecanoyl lysine, N-palmitoyl lysine, N-margaroyl lysine, N-stearoyl lysine, N-n-nonadecanoyl lysine, N-arachidoyl lysine, N-α-linolenoyl lysine, N-stearidonoyl lysine, N-eicosapentaenoyl lysine, N-linoleoyl lysine, N-γ-linolenoyl lysine, N-dihomo-γ-linolenoyl lysine, N-arachidonoyl lysine, N-behenoyl lysine, N-erucoyl lysine, N-capryloyl methionine, N-pelargonoyl methionine, N-capric methionine, N-n-undecanoyl methionine, N-lauroyl methionine, N-n-tridecanoyl methionine, N-myristoyl methionine, N-n-pentadecanoyl methionine, N-palmitoyl methionine, N-margaroyl methionine, N-stearoyl methionine, N-n-nonadecanoyl methionine, N-arachidoyl methionine, N-α-linolenoyl methionine, N-stearidonoyl methionine, N-eicosapentaenoyl methionine, N-linoleoyl methionine, N-γ-linolenoyl methionine, N-dihomo-γ-linolenoyl methionine, N-arachidonoyl methionine, N-behenoyl methionine, N-erucoyl methionine, N-capryloyl phenylalanine, N-pelargonoyl phenylalanine, N-capric phenylalanine, N-n-undecanoyl phenylalanine, N-lauroyl phenylalanine, N-n-tridecanoyl phenylalanine, N-myristoyl phenylalanine, N-n-pentadecanoyl phenylalanine, N-palmitoyl phenylalanine, N-margaroyl phenylalanine, N-stearoyl phenylalanine, N-n-nonadecanoyl phenylalanine, N-arachidoyl phenylalanine, N-α-linolenoyl phenylalanine, N-stearidonoyl phenylalanine, N-eicosapentaenoyl phenylalanine, N-linoleoyl phenylalanine, N-γ-linolenoyl phenylalanine, N-dihomo-γ-linolenoyl phenylalanine, N-arachidonoyl phenylalanine, N-behenoyl phenylalanine, erucoyl phenylalanine, N-capryloyl proline, N-pelargonoyl proline, N-capric proline, N-n-undecanoyl proline, N-lauroyl proline, N-n-tridecanoyl proline, N-myristoyl proline, N-n-pentadecanoyl proline, N-palmitoyl proline, N-margaroyl proline, N-stearoyl proline, N-n-nonadecanoyl proline, N-arachidoyl proline, N-α-linolenoyl proline, N-stearidonoyl proline, N-eicosapentaenoyl proline, N-linoleoyl proline, N-γ-linolenoyl proline, N-dihomo-γ-linolenoyl proline, N-arachidonoyl proline, N-behenoyl proline, N-erucoyl proline, N-capryloyl serine, N-pelargonoyl serine, N-capric serine, N-n-undecanoyl serine, N-lauroyl serine, N-n-tridecanoyl serine, N-myristoyl serine, N-n-pentadecanoyl serine, N-palmitoyl serine, N-margaroyl serine, N-stearoyl serine, N-n-nonadecanoyl serine, N-arachidoyl serine, N-α-linolenoyl serine, N-stearidonoyl serine, N-eicosapentaenoyl serine, N-linoleoyl serine, N-γ-linolenoyl serine, N-dihomo-γ-linolenoyl serine, N-arachidonoyl serine, N-behenoyl serine, N-erucoyl serine, N-capryloyl threonine, N-pelargonoyl threonine, N-capric threonine, N-n-undecanoyl threonine, N-lauroyl threonine, N-n-tridecanoyl threonine, N-myristoyl threonine, N-n-pentadecanoyl threonine, N-palmitoyl threonine, N-margaroyl threonine, N-stearoyl threonine, N-n-nonadecanoyl threonine, N-arachidoyl threonine, N-α-linolenoyl threonine, N-stearidonoyl threonine, N-eicosapentaenoyl threonine, N-cervonoyl threonine, N-linoleoyl threonine, N-γ-linolenoyl threonine, N-dihomo-γ-linolenoyl threonine, N-arachidonoyl threonine, N-behenoyl threonine, N-erucoyl threonine, N-capryloyl tryptophan, N-pelargonoyl tryptophan, N-capric tryptophan, N-n-undecanoyl tryptophan, N-lauroyl tryptophan, N-n-tridecanoyl tryptophan, N-myristoyl tryptophan, N-n-pentadecanoyl tryptophan, N-palmitoyl tryptophan, N-margaroyl tryptophan, N-stearoyl tryptophan, N-n-nonadecanoyl tryptophan, N-arachidoyl tryptophan, N-α-linolenoyl tryptophan, N-stearidonoyl tryptophan, N-eicosapentaenoyl tryptophan, N-linoleoyl tryptophan, N-γ-linolenoyl tryptophan, N-dihomo-γ-linolenoyl tryptophan, N-arachidonoyl tryptophan, N-behenoyl tryptophan, N-erucoyl tryptophan, N-capryloyl tyrosine, N-pelargonoyl tyrosine, N-capric tyrosine, N-n-undecanoyl tyrosine, N-lauroyl tyrosine, N-n-tridecanoyl tyrosine, N-myristoyl tyrosine, N-n-pentadecanoyl tyrosine, N-palmitoyl tyrosine, N-margaroyl tyrosine, N-stearoyl tyrosine, N-n-nonadecanoyl tyrosine, N-arachidoyl tyrosine, N-α-linolenoyl tyrosine, N-stearidonoyl tyrosine, N-eicosapentaenoyl tyrosine, N-linoleoyl tyrosine, N-γ-linolenoyl tyrosine, N-dihomo-γ-linolenoyl tyrosine, N-arachidonoyl tyrosine, N-behenoyl tyrosine, N-erucoyl tyrosine, N-capryloyl valine, N-pelargonoyl valine, N-capric valine, N-n-undecanoyl valine, N-lauroyl valine, N-n-tridecanoyl valine, N-myristoyl valine, N-n-pentadecanoyl valine, N-palmitoyl valine, N-margaroyl valine, N-stearoyl valine, N-n-nonadecanoyl valine, N-arachidoyl valine, N-α-linolenoyl valine, N-stearidonoyl valine, N-eicosapentaenoyl valine, N-linoleoyl valine, N-γ-linolenoyl valine, N-dihomo-γ-linolenoyl valine, N-arachidonoyl valine, N-behenoyl valine and N-erucoyl valine. Preferably these compounds will be in the form of an alkali metal salt, preferably sodium salt. More preferably the N-substituted acyl amino acid is N-stearoyl glutamic acid, more preferably still as an alkali metal salt, especially the sodium salt.

As given above the raw amino acid surfactant would typically be obtained by the reaction of an amino acid and acyl halide under alkaline conditions and in the presence of a polar organic solvent.

Suitably the acyl halide should be a C₆-C₃₀-acyl halide, preferably C₁₂-C₂₂-acyl halide. Although the halide may be any of the halides, for instance fluoride, chloride, bromide or iodide, it is preferred that the halide is the chloride. Suitable examples of C₈-C₂₀-acyl chlorides include capryloyl chloride, pelargonoyl chloride, capric chloride, n-undecanoyl chloride, lauroyl chloride, n-tridecanoyl chloride, myristoyl chloride, n-pentadecanoyl chloride, palmitoyl chloride, margaroyl chloride, stearoyl chloride, n-nonadecanoyl chloride, arachidoyl chloride, α-linolenoyl chloride, stearidonoyl chloride, eicosapentaenoyl chloride, linoleoyl chloride, γ-linolenoyl chloride, dihomo-γ-linolenoyl chloride, arachidonoyl chloride, behenoyl chloride and erucoyl chloride.

Examples of suitable amino acids include glycine, sarcosine, glutamic acid, alanine, arginine, aspartic acid, glutamine, histidine, isoleucine, leucine, lysine, methionine, phenylalanine, proline, serine, threonine, tryptophan, tyrosine and valine. Preferably the amino acid is glutamic acid

The following examples are intended to demonstrate the invention without being limiting.

### Examples

### Examples 1 to 10

N-stearoyl glutamate, disodium salt, was produced as a raw amino acid by the reaction of disodium glutamate and stearoyl chloride at low temperature in an aqueous alkaline medium containing 15 % by weight isopropanol. This is referred to as Raw SG1.

N-stearoyl glutamate, disodium salt, was produced by feeding stearoyl chloride to a stirred aqueous solution of sodium glutamate at pH > 10 and containing 15 wt. -% isopropanol at temperatures < 20 °C in excess of sodium glutamate and without exceeding a solids content of 36 wt%.

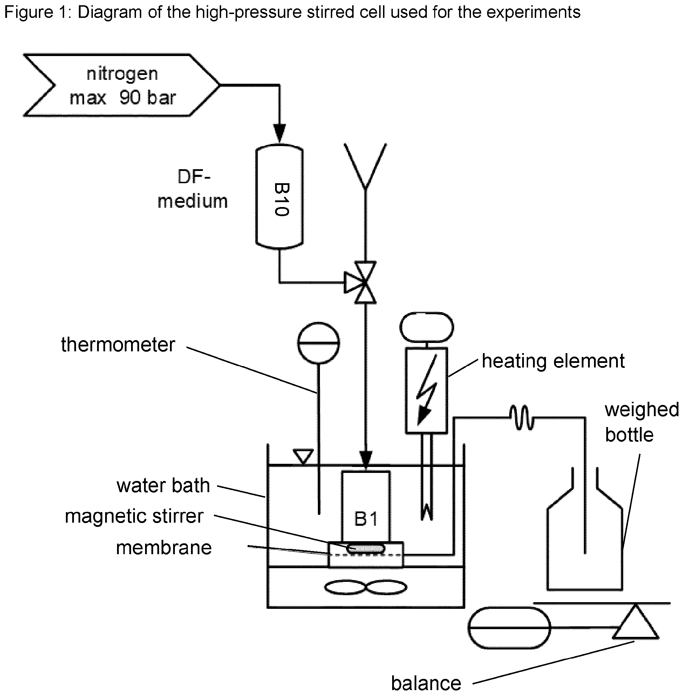

For the experiments 1-10 a high-pressure stirred cell unit (see Figure 1) was used. The apparatus consists of the retentate cell B1 mounted in a thermostatically controlled water bath and the vessel B10 for Raw SG1 feed or diafiltration medium. The membrane is installed at the bottom of the cell B1 (as indicated). The liquid in cell B1 is stirred by a magnetic stirrer. By applying a pressure up to 25 bar to the vessel B10, the permeate is pressed through the membrane and collected in a bottle placed on a balance. In the experiments 10 ultrafiltration membranes from three suppliers were tested as indicated in Table 1.

The parameters of the experiments are compiled in Table 1 and the results of the experiments are shown in Table 2.

**Table 1: Experiments in the high-pressure stirred cell temperature 65°C, feed material Raw SG1, provided undiluted having 36% solids.**

| example | membrane | membrane material | MWCO [kD] / pore size [nm] | Trans membrane pressure TMP [bar] |
|---|---|---|---|---|
| example 01 | Suez MW | polyacrylonitrile (PAN) | 50 kD | 5-7 |
| example 02 | Suez PW | polyethersulfone (PES) | 20 kD | 5-10 |
| example 03 | CUT 10 kD | polyethersulfone (PES) | 10 kD | 10 |
| example 04 | CUT 5 kD | polyethersulfone (PES) | 5 kD | 10 |
| example 05 | Microdyn Nadir UH004 | polyethersulfone hydrophilic (PESH) | 4 kD | 20 |
| example 06 | Microdyn Nadir UP005 | polyethersulfone (PES) | 5 kD | 10-20 |
| example 07 | Microdyn Nadir UP010 | polyethersulfone (PES) | 10 kD | 5-10 |
| example 08 | Microdyn Nadir UP020 | polyethersulfone (PES) | 20 kD | 10 |
| example 19 | Microdyn Nadir UH030 | polyethersulfone hydrophilic (PESH) | 30 kD | 10 |
| example 10 | Microdyn Nadir UH050 | polyethersulfone hydrophilic (PESH) | 50 kD | 25 |

**Table 2 Results of the experiments in the high-pressure stirred cell temperature 65 °C, Raw SG1 feed, provided undiluted**

| Ex. | Diafiltration factor DF | Av. flux [kg/m²/h] | Concentration of NaCl | | | Concentration of isopropanol | | | Concentration of stearoyl glutamate | | | Stearic acid |
|---|---|---|---|---|---|---|---|---|---|---|---|---|
| | | | Raw SG1 feed [kg/k g] | Retentate [kg/kg] | Permeate [kg/kg] | Raw SG1 feed [kg/k g] | Retentate [kg/kg] | Permeate [kg/kg] | Raw SG1 feed [kg/kg] | Retentate [kg/kg] | Permeate [kg/kg] | Raw SG1 feed [kg/kg] |
| 1 | 3.05 | 17 | 3.709 % | 0.019 % | 1.2% | 16.1 % | 0.7% | 4.4% | 29.800 % | 11.900 % | 5.6% | <0,1 % |
| 2 | 2.97 | 20 | 3.709 % | 0.018 % | 1.2% | 16.1 % | 1.0% | 3.4% | 29.800 % | 12.100 % | 5.5% | <0,1 % |
| 3 | 3.04 | 20 | 3.709 % | 0.018 % | 1.2% | 16.1 % | 0.9% | 3.3% | 29.800 % | 12.300 % | 5.7% | <0,1 % |
| 4 | 3.00 | 42 | 3.709 % | 0.055 % | 1.5% | 16.1 % | 0.7% | 2.4% | 29.800 % | 8.400% | 6.9% | <0,1 % |
| 5 | 1.59 | 4 | 3.709 % | 0.077 % | 2.2% | 16.1 % | 4.0% | 6.7% | 29.800 % | 20.200 % | 5.0% | <0,1 % |
| 6 | 1.26 | 4 | 3.709 % | 0.150 % | 2.7% | 16.1 % | 5.2% | 4.3% | 29.800 % | 21.600 % | 5.4% | <0,1 % |
| 7 | 2.93 | 14 | 3.709 % | 0.014 % | 1.2% | 16.1 % | 1.5% | 4.6% | 29.800 % | 15.000 % | 4.6% | <0,1 % |
| 8 | 2.96 | 22 | 3.709 % | 0.018 % | 1.2% | 16.1 % | 0.8% | 4.6% | 29.800 % | 10.800 % | 6.0% | <0,1 % |
| 9 | 2.97 | 216 | 3.709 % | 0.130 % | 1.1% | 16.1 % | 0.9% | 2.6% | 29.800 % | 2.700% | 9.0% | <0,1 % |
| 10 | 3.02 | 86 | 3.709 % | 0.116 % | 1.2% | 16.1 % | 1.1% | 1.7% | 29.800 % | 3.200% | 9.0% | <0,1 % |

### Examples 11-15

N-stearoyl glutamate, disodium salt, was produced as a raw amino acid surfactant by the reaction of disodium glutamate and stearoyl chloride in an aqueous alkaline medium containing isopropanol. This is referred to as Raw SG2. The composition of the Raw SG2 was sodium chloride 3.7 wt%, isopropanol 7.0 wt%, Na-stearyl-glutamate 25.4 wt%, Na-glutamate 3.1 wt%, and stearic acid 2.6 wt%, with the remainder being water.

N-stearoyl glutamate, disodium salt, was produced by feeding stearoyl chloride to a stirred aqueous solution of sodium glutamate at pH > 10 and containing 7 wt. -% isopropanol at temperatures < 20 °C in excess of sodium glutamate and without exceeding a solids content of 36 wt%.

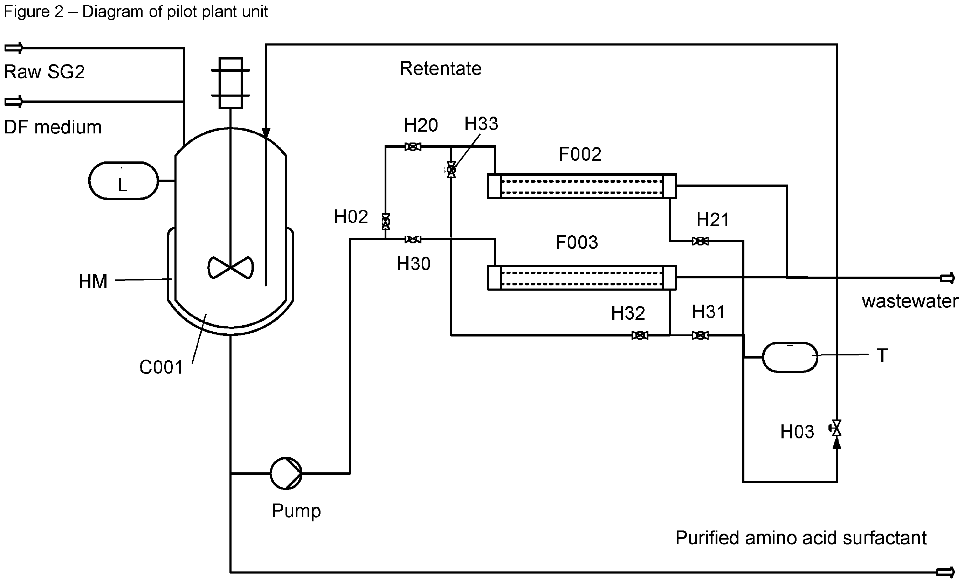

The pilot plant consisted of a stainless-steel vessel C001, pressure pump (P) and the membrane units F002 and F003 which are connected according to Figure 2. The 1.5 m³ vessel C001 is equipped with a stirrer, a heating mantle (HM), level indicator (L) and a temperature indicator (T). The pressure pump (P) enables feeding of the diluted Raw SG2 into the membrane unit F003 and/or F002 and can be operated automatically according to Figure 2. The stainless-steel membrane units F003 and F002 are suitable to accommodate two 4-inch spiral wound membranes and can be operated in parallel and in series mode, employing the appropriate combination of valves H02, H20, H21, H30, H31, H32, H33 illustrated in Figure 2.

For the pilot plant experiments the stirred vessel C001 and the filtration unit were used. The vessel C001 was equipped with a retentate return pipe and a supply for diluted Raw SG2 synthesis product and diafiltration (DF) medium shown in Figure 2. The diafiltration (DF) medium was deionised water. The filling volume of the vessel C001 was 1500 liters.

In Examples 11-15 the filter membrane used was spiral wound modules SPIRA-CEL^{®} OY UP010 4040C, 46 mil Spacer, membrane area 6.0 m² from Microdyn-Nadir having a nominal molecular weight cut off or 10,000 Da.

Table 3 provides an overview of the pilot plant experiments of Examples 11-15.

**Table 3: process parameters of the pilot plant experiments**

| Example | 011 | 012 | 013 | 014 | 015 |
|---|---|---|---|---|---|
| membrane area [m²] | 6 | 6 | 12 | 12 | 12 |
| dilution of feed | 1:5 | 1:2 | 1:2.7 | 1:1.5 | 1:2 |
| dilution_factor⁻¹ | 0.17 | 0.33 | 0.27 | 0.40 | 0.33 |
| temperature | 59°C | 59°C | 42°C | 40°C | 45°C |
| surface velocity | 0.3 / 0.4 m/s | 0.5 m/s | 0.5 m/s | 0.5 m/s | 0.5 m/s |
| crossflow | 2.8 / 3.7 m³/h | 4.6 m³/h | 4.6 m³/h | 4.6 m³/h | 4.6 m³/h |
| average TMP | 9.9 bar | 9.8 bar | 9.5 bar | 9.6 bar | 9.6 bar |
| undiluted synthesis product | 500 kg | 500 kg | 400 kg | 500 kg | 600 kg |
| diluted feed start filling | 1500 kg | 1500 kg | 1500 kg | 1300 kg | 1500 kg |
| diluted feed added in process | 1490 kg | | | | 200 kg * |
| initial diafiltration step, DF1 | | | 0.5 | 0.5 | 0.5 |
| initial concentration step, CF1 (mode) | 2.0 (fed batch) | 1.5 (batch) | 1.4 (batch) | 1.2 (batch) | 1.5 (batch) |
| diafiltration step, DF2 | 3.0 | 2.8 | 2.3 | 2.1 | 2.5 |
| final concentration step, CF2 | 2.1 | 1.5 | 1.7 | 2.2 | 1.4 |
| total process, DF | 4.0 | 2.8 | 2.8 | 2.5 | 3.0 |
| total process, CF | 4.1 | 2.2 | 2.5 | 2.7 | 2.1 |

| | | | | | |
|---|---|---|---|---|---|
| * This feed material was 1:1 diluted. | | | | | |

Initially the vessel C001 was filled with 1500 kg of diluted synthesis product.

In the example 011 the synthesis product Raw SG2 was diluted 1:5 with deionized water (DI-water), to keep the isopropanol concentration in the mixture below 1.4 wt% and the flashpoint above 80°C. In this example the remaining feed material (about 1500 kg) was fed in the vessel by a fed-batch concentration process. In the example 016 the feed material (200 kg) was filled in the vessel C001 after an initial concentration step.

In the example 013 to 015 the filtration process started with an initial diafiltration step DF1 to reduce the concentration of isopropanol in the retentate. Then the level in the vessel C001 was lowered by an initial concentration step. Examples 011 and 012 did not employ an initial diafiltration step DF1.

In all examples sodium chloride and isopropanol were removed by the following diafiltration step. At the end, in the final concentration step the purified product was concentrated to its final content of Na-stearyl-glutamate. Please find the diafiltration factors DF and the concentration factors of the total process in Table 3.

The filtration temperature was 40°C to 60°C. The crossflow of the example 011 was 2.8 m³/h to 3.7 m³/h. In the example 012 to 015 a crossflow of 4.6 m³/h was used. The inlet pressure of the first module was kept constant at 10 bar. The transmembrane pressure TMP was 9.5 bar to 9.9 bar.

**Table 4: Overview of flux in the pilot plant examples; membrane module: SPIRA-CEL^{®} OY UP010 4040C, 46 mil Spacer; Raw SG2 feed material.**

| Example | 011 | 012 | 013 | 014 | 015 |
|---|---|---|---|---|---|
| start dilution of feed | 1:5 | 1:2 | 1:2.7 | 2:3 | 1:2 |
| start dilution_factor⁻¹ | 0.17 | 0.33 | 0.27 | 0.40 | 0.33 |
| average TMP [bar] | 9.9 | 9.8 | 9.5 | 9.6 | 9.6 |
| temperature [°C] | 59 | 59 | 42 | 40 | 45 |
| surface velocity [m/s] | 0.3 / 0.4 | 0.5 | 0.5 | 0.5 | 0.5 |
| crossflow [kg/h] | 2800 / 3700 | 4600 | 4600 | 4600 | 4600 |
| filtration time [h] | 19 | 16 | 14 | 32 | 21 |
| average flux DF1 [kg/m²/h] | | | 53 | 37 | 30 |
| average flux AK1 [kg/m²/h] | 80 | 59 | 47 | 31 | 24 |
| average flux DF2 [kg/m²/h] | 66 | 48 | 32 | 15 | 22 |
| average flux AK2 [kg/m²/h] | 31 | 15 | 7 | 4 | 6 |
| average total flux [kg/m²/h] | 60 | 41 | 25 | 11 | 19 |
| maximum flux [kg/m²/h] | 120 | 73 | 60 | 39 | 62 |

The results presented in Table 5 below show that sodium chloride and isopropanol could easily be removed. Examples 011, 012, 014 and 015 show no sodium chloride was found in the final retentate. In example 013 the sodium chloride content in the file retentate was reduced to 0.2 wt.%. The concentration of isopropanol in the final retentate was below 0.4 wt.% for all the examples. Sodium stearoyl glutamate was retained in the retentate in all examples no less than 16.9 wt.% and up to 23.4 wt.%. Sodium glutamate was removed from the retentate to levels below 0.1 wt.%.

**Table 5: Concentration in the final retentate, analytical values**

| experiment | NaCl [kg/kg] | IPA [kg/kg] | Na-stearyl-glutamate [kg/kg] | Na-glutamate [kg/kg] | Stearic acid [kg/kg] |
|---|---|---|---|---|---|
| 011 | 0.0% | 0.2% | 16.9% | <0.1% | 1.5% |
| 012 | 0.0% | 0.4% | 19.2% | <0.1% | |
| 013 | 0.2% | 0.3% | 17.6% | <0.1% | 1.5% |
| 014 | 0.0% | 0.3% | 23.4% | <0.1% | 1.7% |
| 015 | 0,0% | 0,4% | 20,6% | <0,1% | 1,8% |

The permeate produced in examples 11 - 15 had isopropanol concentrations below 2 wt.% and hence the flashpoint of the permeate was higher than 60°C.

The examples show that the amino acid surfactant can be produced with high purity containing very low contents of impurities, notably inorganic salt and amino acid e.g. sodium chloride and sodium glutamate. Further the examples illustrate a high filtration flow rate. Furthermore, the permeate contains relatively low levels of polar organic solvent which means that it can be disposed of as wastewater not having a low flashpoint.

## Claims

1. A method for producing a purified amino acid surfactant from a raw amino acid surfactant containing impurities, comprising the steps of
(i) providing the raw amino acid surfactant; and
(ii) subjecting the raw amino acid surfactant to an ultrafiltration stage; and
(iii) collecting the so produced purified amino acid surfactant,
wherein the ultrafiltration stage comprises a filter membrane having a separation limit molecular cut-off from 1000 g/mol to 100,000 g/mol, preferably from 4000 g/mol to 50,000 g/mol, more preferably from 5000 g/mol to 30,000 g/mol.

2. The method according to claim 1, wherein the impurities contained in the raw amino acid surfactant comprise an inorganic salt, a polar organic solvent and optionally an amino acid.

3. The method according to claim 1 or claim 2, wherein the filter membrane is constructed from any of the materials selected from the group consisting of polyacrylonitrile, polyethersulfone, hydrophilized polyethersulfone and any combinations of these materials thereof.

4. The method according to any preceding claim, wherein the raw material amino acid surfactant is comprised in an aqueous medium, preferably which aqueous medium has a pH of greater than 4.0.

5. The method according to any preceding claim, wherein the raw amino acid surfactant is diluted with an aqueous dilution medium, preferably by an amount of from 1:10 to 3:1.

6. The method according to any preceding claim, wherein the ultrafiltration stage comprises crossflow filtration.

7. The method according to claim 6, wherein the raw amino acid surfactant is in an aqueous medium which is flowed through the ultrafiltration stage forming a permeate and a retentate, in which impurities are removed through the permeate and amino acid surfactant is preferentially retained in the retentate, which retentate is recirculated in a recirculation loop.

8. The method according to claim 7, wherein the raw amino acid surfactant in the aqueous medium is subjected to at least one diafiltration step, in which a diafiltration medium is fed into the recirculation loop at substantially the same rate that permeate flows from the ultrafiltration stage.

9. The method according to claim 8, wherein the at least one diafiltration step has an overall diafiltration factor from 1 to 10, preferably from 2 to 6.

10. The method according to any of claims 7 to 9, wherein the raw amino acid surfactant in the aqueous medium is subjected to at least one concentration step, in which no dilution medium is fed into the recirculation loop or dilution medium is fed into the recirculation loop at a lower rate than the rate of permeate that flows from the ultrafiltration stage.

11. The method according to claim 10, wherein the raw amino acid surfactant in the aqueous medium is subjected to a combination of diafiltration and concentration carried out in series.

12. The method according to any of claims 7 to 11, wherein diafiltration and/or concentration steps are carried out in sequence comprising
a) optionally initial diafiltration (D1);
b) optional initial concentration (C1);
c) optional diafiltration (D2); and
d) optional final concentration (C2),
provided that at least one of steps a or c and at least one of steps b or d are included, preferably where at least steps b) to d) are included, more preferably where all steps a) to d) are included.

13. The method according to claim 12, wherein the optional initial diafiltration (D1) is carried out with the diafiltration factor (DF1) from 0.01 to 3.0, preferably from 0.3 to 1.0.

14. The method according to claim 12 or claim 13, wherein the initial concentration (C1) is carried out with a concentration factor (CF1) from 1.01 to 10.0, preferably from 1.3 to 2.0.

15. The method according to any of claims 12 to 14, wherein the diafiltration (D2) is carried out with a diafiltration factor (DF2) from 0.01 to 10.0, preferably from 1.5 to 5.0.

16. The method according to any of claims 12 to 15, wherein the final concentration (C2) is carried out with a concentration factor (CF2) from 1.1 to 4.0, preferably from 1.2 to 3.0.

17. The method according to any preceding claim, wherein the method is carried out in batch or fed batch operation.

18. The method according to any preceding claim, wherein the amino acid surfactant is in the form of an alkali metal salt, preferably sodium salt.

19. The method according to any preceding claim, wherein the amino acid surfactant is an N-substituted C₈-C₃₀-acyl amino acid, preferably N-substituted C₁₂-C₂₀-acyl amino acid.

20. The method according to any preceding claim, wherein the raw amino acid surfactant has been obtained by the reaction of an amino acid and acyl halide under alkaline conditions and in the presence of an organic polar solvent.

21. The method according to any preceding claim, wherein the amino acid surfactant is derived from any amino acid, desirably an amino acid selected from the group consisting of glycine, sarcosine, glutamic acid, alanine, arginine, aspartic acid, glutamine, histidine, isoleucine, leucine, lysine, methionine, phenylalanine, proline, serine, threonine, tryptophan, tyrosine and valine, preferably glutamic acid
